# EUROPEAN PATENT APPLICATION

(11) **EP 3 988 675 A1**
(43) Date of publication of application: **27.04.2022**
(21) Application number: 20202996.3
(22) Date of filing: 21.10.2020
(51) Int. Cl.: C12Q 1/6886

(54) **METHOD FOR DIFFERENTIAL DIAGNOSIS OF PROSTATE DISEASE AND MARKER FOR DIFFERENTIAL DIAGNOSIS OF PROSTATE DISEASE AS WELL AS KIT THEREFOR**

(71) Applicant: PRIVATE UNIVERSITÄT WITTEN/HERDECKE GMBH, 58448 Witten (DE)
(72) Inventor: KAUFMANN, Michael, 58300 Wetter (DE); SAVELSBERGH, Andreas, 45133 Essen (DE); KLINGER, Claudia, 58453 Witten (DE); MARKERT, Lukas, 58453 Witten (DE); HOLDMANN, Jonas, Witten 58452 (DE)
(74) Representative: Gramm, Lins & Partner Patent- und Rechtsanwälte PartGmbB

(57) **Abstract**

The present invention relates in a first aspect to a method for differential diagnosis of prostate cancer (PCa) in a subject as well as a method for screening for prostate cancer in a subject and, in addition, to a method for screening benign prostatic hyperplasia (BPH) in a subject. The method according to the present invention is based on determining the level or amount of the expression of hsa-piR-018849 nucleic acid molecules in a sample of said subject and based on the determined level or amount of expression of said nucleic acid, allowing differential diagnosis or identification of PCa or BPH respectively. Further, the present invention relates to a method for monitoring the development, progress or lapse of prostate cancer as well as to a method for a stratification of a therapeutic regimen of a subject being afflicted or suspected to be afflicted with or being at risk of developing PCa. In addition, a test kit for diagnosing prostate cancer or benign prostate disease, disorder or condition in a subject is provided. Moreover, the use of hsa-piR-018849 nucleic acid is a marker for differential diagnosis of prostate disease, disorder or condition, in particular, allowing differentiation between PCa and PBH is provided. In an embodiment, determination is supplemented by determining the level or amount of expression of further nucleic acids as described.

## Description

The present invention relates in a first aspect to a method for differential diagnosis of prostate cancer (PCa) in a subject as well as a method for screening for prostate cancer in a subject and, in addition, to a method for screening benign prostatic hyperplasia (BPH) in a subject. The method according to the present invention is based on determining the level or amount of the expression of hsa-piR-018849 nucleic acid molecules in a sample of said subject and based on the determined level or amount of expression of said nucleic acid, allowing differential diagnosis or identification of PCa or BPH respectively. Further, the present invention relates to a method for monitoring the development, progress or lapse of prostate cancer as well as to a method for a stratification of a therapeutic regimen of a subject being afflicted or suspected to be afflicted with or being at risk of developing PCa. In addition, a test kit for diagnosing prostate cancer or benign prostate disease, disorder or condition in a subject is provided. Moreover, the use of hsa-piR-018849 nucleic acid is a marker for differential diagnosis of prostate disease, disorder or condition, in particular, allowing differentiation between PCa and PBH is provided. In an embodiment, determination is supplemented by determining the level or amount of expression of further nucleic acids as described.

### Prior art

The prostate is an accessory sex gland. The prostate is located on the underside of the bladder enclosing the first part of the urethra. Based on histological and clinical parameters, 3 concentric zones can be distinguished, namely, the central/inner zone, the transition zone and the peripheral/outer zone. The prostate produces exocrine about one third of the ejaculate volume and thus serves the survival and mobility of the sperm.

With increasing age, the tissue of the transition and central zone surrounding the urethra tends to benign enlargement which is also known and described as benignant prostatic hyperplasia (BPH). About 75 % of men between the ages of 60 to 69 are affected by BPH. Moreover, autopsy studies revealed that the prevalence increases up to 86 % in people over 80 years of age.

BPH is also sometimes called prostate enlargement representing a noncancerous increase in size of the prostate gland. Symptoms may include frequent urination, trouble starting to urinate, weak stream, inability to urinate or loss of bladder control. Although several risk factors are described in the prior art, the cause remains unclear. A diagnosis is typically based on symptoms and examination after ruling out other possible causes. BPH is the most common cause of lower urinary tract symptoms. Various types of cells undergo hyperplasia in BPH, including the glandular epithelial cells and stromal cells. Differential diagnosis of BPH is difficult since various stages of BPH can show very similar symptoms as the prostate cancer, including reduction of urinary stream, urinary tensions of the urinary tract. However, treatment regimen and outcome is essentially different.

Prostate cancer is the most common cancer and the third most frequent cause of male cancer deaths in Germany and worldwide. Autopsies revealed that 59 % of people over 79 years old men had PCa, probably unnoticed by the individual. In 95 % it is an adenocarcinoma, mostly a peripheral adenocarcinoma.

Today for diagnostic differentiation between these two types of diseases, BPH and PCA, basically digital rectal examination, transrectal ultrasound and PSA levels in blood are used. Further methods for differential diagnosis include CT and MRI diagnosis, which are even more expensive and time consuming. Since these methods are quite error-prone and/or resourceful, the gold standard for the diagnosis of different types of prostate disease, disorder or conditions is the transrectal punch biopsy. However, main disadvantage of this type of diagnosis is the quite painful procedure which additionally carries risk for the patient.

For prostate cancer classifications are typically based on the staging system TNM (classification of malign tumours) and Gleason score, which distinguishes the risk groups. There are five degrees of malignancy according to Gleason, the primary growth profile of prostate cells and the second most common profile are scored by a point system. Addition of the two values give the final score, resulting in scores between 2 and 10, accordingly. Based on this final score it is possible to estimate the extend of malignancy within a biopsy obtained from a patient.

Until today it is believed that the needle prostate biopsy is essential to catch the pathological presence of prostate cancer and, in addition, to allow differential diagnosis of prostate cancer vs. BPH or other types of prostate diseases. The methods described so far are not sufficient to allow a differentiation with high specificity. For example, PSA is known as a marker, however, PSA testing demonstrated low specificity and very low sensitivity, thus, restricting its diagnostic significance. With respect to PSA, various methods have been described to improve the accuracy of diagnosis, however, failed until today.

Moreover, other markers have been described to allow discrimination between two different types of prostate diseases. For example, Adel mosa Ahmed Al-Rekabi, J. Pharm. Sci. & Res., 2018, 10(8), 1885-1889, identifies a microRNA, miR 1825, allegedly allowing early diagnosis of prostate tumour and having good screening properties for prostate cancer further allowing a discrimination against BPH. The test is based determining the level or amount of urine miRNA 1825 by PCR-method. Other miRNAs have been described as potential urinary biomarker candidates for accurate detection of prostate cancer among BPH patients.

Wang Y., et al., Oncology Research and Treatment, DOI: 10.1159/000504606, 2019, identify miR-1231 being downregulated in prostate cancer with prognostic and functional implications.

Taha A Haj-Ahmad, et al., Journal of Cancer, 2014, 5(3), 182-191, describe the potential urinary miRNA biomarker candidates for the accurate detection of prostate cancer among benign BPH patients. Various miR nucleic acids are disclosed allegedly having biomarker potential.

Additional studies have been conducted by Cochetti, et al., 2016, OncoTargets and Therapy, 16:9, 7545-7553, referring to different levels of serum microRNAs in prostate cancer and BPH and evaluating the potential diagnostic and prognostic role.

Song C-J., et al., J Cell Biochem., 2018, 119, 2763-2786, refers to the potential of microRNAs as human prostate cancer biomarkers providing a meta-analysis of related studies.

Piwi genes were identified as regulatory proteins responsible for stem cell and germ cell differentiation. Piwi is an abbreviation of P-element Induced Wimpy testis in Drosophila and the proteins are highly conserved RNA-binding proteins and are present in both plants and animals. In addition, Piwi-interacting RNA (piRNA) represent the largest class of small non-coding RNA molecules expressed in animal cells. PiRNAs form RNA-protein complexes through interactions with piwi-subfamily proteins. PiRNAs are mostly created from loci that function as transposon traps and provide a RNA-mediated adaptive immunity against transposon expansions and invasions. They are distinct from microRNA (miRNA) in size (typically they are larger with the size of 26 - 31 nt), lack of sequence conservation, increased complexity and independence of Dicer for biogenesis at least in animals. It has been shown that piRNA has a role in RNA silencing via the formation of a RNA induced silencing complex (RISC).

Weng W., et al., Biochim Biophys Acta Rev. Cancer, 2019, 1871(1), 160-169, refers to piwi-interacting RNAs (piRNAs) and cancer: emerging biological concepts and potential clinical implications.

However, there is still the problem of sufficient specificity and sensitivity combined with fast and easy handling, including non-invasive steps.

### Brief description of the present invention

In a first aspect the present invention relates to a method for differential diagnosis or for identification of prostate cancer in a subject suspected to suffer from prostate cancer or other type of prostate disease, disorder or condition, comprising the steps of:
a) providing a sample of said subject to be diagnosed;
b) determining the level or amount of the expression of hsa-piR-018849 nucleic acid in said sample;
differential diagnosing or identifying prostate cancer or other type of prostate disease, disorder or condition, in particular, a benign prostate disease, disorder or condition based on the level or amount of the hsa-piR-018849 nucleic acid determined in step b). In addition, based on determining the level or amount of expression of hsa-piR-018849 nucleic acid, a screening method for prostate cancer in a subject is provided as well as a method for screening for benignant prostatic hyperplasia (BPH) in a subject suspected to suffer from a prostate disease, disorder or condition.

Furthermore, a method for monitoring the development, progress or lapse of prostate cancer a subject is afflicted with, suspected to be afflicted with or having a risk of developing prostate cancer is provided based on determining the level or amount of expression of hsa-piR-018849 nucleic acid in a sample of said subject in a first time point and, optionally, a second time point and comparing said level or amount determined in at least a first time point with the level or amount of said nucleic acid either detected at the second time point or compared to a reference value of normal samples.

Further, a method for the stratification of the therapeutic regimen of a subject being afflicted with, suspected to be afflicted with or being at risk of developing prostate cancer, comprising the steps of
a) determining the level or amount of expression of hsa-piR-018849 nucleic acid in a sample of said subject, an
b) determining a therapeutic regimen of said subject taking into account or based on that level or amount of the expression of hsa-piR-018849 nucleic acid relative to the level or amount of the expression of hsa-piR-018849 nucleic acid in a normal sample.

In addition, the present invention relates to a test kit for diagnosing prostate cancer or benignant prostate disease, disorder or condition in a subject, in particular, BPH, said kit comprises means for determining the expression level or amount of at least hsa-piR-018849 and, optionally, further means for determining the expression level or amount of at least one of hsa-piR-019324, hsa-miR-15a-5p, hsa-miR-425-3p, hsa-miR-150-5p, hsa-miR-3126-3p, hsa-miR-324-5p or hsa-miR-6078 and instructions on how to use said test kit for a method according to any one of the claims 1 to 11.

Finally, the use of the hsa-piR-018849 as a marker for differential diagnosis of prostate disease, disorder or condition is provided. This marker may be used in combination with at least one of hsa-piR-019324, hsa-miR-15a-5p, hsa-miR-425-3p, hsa-miR-150-5p, hsa-miR-3126-3p, hsa-miR-324-5p or hsa-miR-6078.

### Brief description of the drawings

Figure 1 shows the ROC curves of the piRNA according to the present invention (1a) and of the second piRNA which may be used in combination (1b).
Figure 2 provides the ROC curves of the miRNAs miR 15a-5p, 150-5p, 425-3p, 324-5p, 3126-3p and 6078.

### Detailed description of the present invention

The present inventors aim in providing a fast, reliable and non-invasive method allowing differential diagnosis of prostate cancer and benignant prostatic hyperplasia. Generally, the present inventors recognized that the hsa-piR-018849 nucleic acid reasonably presents a suitable biomarker for diagnosing prostate cancer. Hence, in a first aspect a method for differential diagnosis or for identification of prostate cancer in a subject suspected to suffer from prostate cancer or other type of prostate disease, disorder or condition is provided, comprising the steps of:
a) providing a sample of said subject to be diagnosed;
b) determining the level or amount of the expression of hsa-piR-018849 nucleic acid in said sample;
differential diagnosing or identifying prostate cancer or other type of prostate disease, disorder or condition, in particular, a benign prostate disease, disorder or condition based on the level or amount of the hsa-piR-018849 nucleic acid determined in step b).

That is, the present inventors recognized that the specific hsa-piR-018849 nucleic acid (SEQ ID No. 1) is a biomarker suitable for differential diagnosis of prostate disease, disorder or condition. In particular, this biomarker allows the differential diagnosis of prostate cancer and BPH. It is identified that a decrease of the expression level or amount of the hsa-piR-018849 nucleic acid is indicative of prostate cancer while in BPH, the expression level or amount is similar to the expression level or amount of said molecule in subjects not afflicted with a prostate disease, disorder or condition.

A "subject" in the context of the present invention is preferably a mammal. A mammal can be a human, non-human primate, mouse, rat, dog, cat, horse or cow but are not limited to these species. A subject can be a male or female. It is preferred that the subject is human. A subject can be one who has been previously with or identified as suffering from a prostate disease, disorder or condition and, optionally, not having already undergone treatment of said disease or disorder. A subject can also be one who has been diagnosed for a specific prostate disease, disorder or condition but shows improvement in the disease as the result of receiving one or more treatments of said disease or disorder. Moreover, a subject may also be one who has not been previously diagnosed or identified as suffering from prostate disease, disorder or condition. A subject can also be one who is suffering from or at risk of developing prostate disease, disorder or condition, e. g. due to genetic predisposition.

The term "determining" as used herein refers to assessing the presence, absence, quantity, level or amount of either a given substance within a clinical or subject derived sample. In particular, the term "determining" refers to assessing physically the level or amount of a given nucleic acid sequence.

As used herein, the term "comprise" or "comprising" as well as the terms "contain" or "containing" include the embodiments of "consists of" or "consisting of".

In the context of the present invention, the term "sample" refers to a biological sample isolated from the subject which can include body fluid or body tissue. Preferably the sample is a body fluid sample or sample of the body fluid being derived from urine or blood, like whole blood, serum or plasma. It is preferred, that the sample is a non-invasive obtained sample.

In the context of the present invention, the term "reference value" refers to an index value or a value derived from one or more prostate cancer risk predictions algorithms or computed indices a value derived from at least subject with the same disease or a value derived from the subject diagnosed with or identified of suffering from a specific prostate disease, disorder or condition. In particular, e. g. in case of the method for diagnosing prostate cancer, the reference values obtained from subjects not afflicted with prostate cancer or preform cells and, in addition, the reference value represents a range from an index obtained from at least two samples collected from a subject not afflicted with the prostate disease, disorder or condition.

As used herein "differential diagnosis of prostate cancer" refers to diagnosis or identification of suffering from prostate cancer of a subject suspected to suffer from prostate cancer or other type of prostate disease, disorder or condition including benignant prostate disease.

In a further aspect, the present invention relates to a method for screening for prostate cancer in a subject comprising the steps of
a) providing a sample of said subject;
b) determining the level or amount of the expression of hsa-piR-018849 nucleic acid in said sample;
c) comparing said level or amount of the expression of hsa-piR-018849 nucleic acid to a reference value from a normal sample(s) whereby a decrease of the expression level or amount is indicative of prostate cancer.

As noted, a decrease in the expression level or amount of the hsa-piR-018849 nucleic acid is indicative of prostate cancer in a subject suspected or being afflicted with a prostatic disease, disorder or condition.

Moreover, the present invention relates to a method for screening for benign prostatic hyperplasia (BPH) in a subject suspected to suffer from a prostate disease, disorder or condition, comprising the steps of
a) providing a sample of said subject;
b) determining the level or amount of the expression of hsa-piR-018849 nucleic acid in said sample;
c) comparing said level or amount of the expression of hsa-piR-018849 nucleic acid determined in step b) to a reference value and excluding prostate cancer or other malignant prostate diseases, disorders or conditions.

This is, in a subject suspected to suffer from a prostate disease, disorder or condition, it is difficult to differentiate between prostate cancer and benignant prostatic hyperplasia. Based on the expression level or amount of the hsa-piR-018849 nucleic acid, a differential diagnosis or screening for either prostate cancer or benignant prostatic hyperplasia is possible. While the level or amount of expression of hsa-piR-018849 nucleic acid is essentially not different in patients suffering from BPH compared to normal reference values, the expression level or amount of hsa-piR-018849 nucleic acid is decreased and, thus, indicative for prostate cancer.

In a further aspect, the present invention relates to a method for monitoring the development, progress or lapse of prostate cancer in a subject afflicted with, suspected to be afflicted with or having a risk of developing prostate cancer, comprising the steps of
a) determining the level or amount of expression of hsa-piR-018849 nucleic acid in a sample of said subject at a first time point, and
b) optionally, determining the level or amount of said nucleic acid, at a second time point, and
c) comparing the level or amount of expression of said nucleic acid determined in step a) to the level or amount of said nucleic acid detected in step b) or to a reference value of a normal sample(s).

Beside the above possible use of the present biomarker hsa-piR-018849 nucleic acid in differential diagnosis of prostate cancer and BPH as well as for screening prostate cancer and BPH in a subject, respectively, this biomarker is also suitable for monitoring the developmental progress or lapse of prostate cancer in a subject. Namely, based on the level or amount of expression of the nucleic acid hsa-piR-018849, it is possible to identify the progress or lapse of prostate cancer accordingly.

Further, the present invention provides a method for the stratification of the therapeutic regimen of a subject being afflicted with, suspected to be afflicted with or being at risk of developing prostate cancer, comprising the steps of
a) determining the level or amount of expression of hsa-piR-018849 nucleic acid in a sample of said subject, and
b) determining a therapeutic regimen of said subject taking into account or based on that level or amount of the expression of hsa-piR-018849 nucleic acid relative to the level or amount of the expression of hsa-piR-018849 nucleic acid in a normal sample.

Namely, the present biomarker is suitable for stratifying the therapeutic regimen of a treatment of prostate cancer based on the level or amount of the expression of said nucleic acid. Further, it is possible to monitor treatment success as well as favourable progress or failure of treatment.

In an aspect, the present invention refers to the method wherein a low level or amount of expression of hsa-piR-018849 nucleic acid in said sample of a subject compared to a reference sample which is a normal sample providing a control relative to the test sample of said subject, is indicative for being afflicted with prostate cancer or being at risk of developing prostate cancer or for the risk of developing relapse of prostate cancer. In addition, the low level or amount of expression of the nucleic acid according to the present invention allows stratification of the therapeutic regimen, namely, deciding on the treatment including chemotherapy, surgery and combinations thereof.

In contrast, in case the level or amount of the expression of hsa-piR-018849 nucleic acid is not significantly different to the level or amount in a reference sample obtained e. g. from a normal sample is indicative that the subject is not afflicted with prostate cancer or being at risk of developing prostate cancer but a benignant prostate disease is present when other signs for prostate disease are given. For example, the subject suffers from benignant prostate hyperplasia (BPH) in combination with other signs of prostate disease, accordingly.

Further, the present invention includes embodiments wherein beside determining the level or amount of expression of hsa-piR-018849 nucleic acid also the level or amount of expression of hsa-piR-019324 nucleic acid (SEQ ID No. 2) can be determined. Combining the biomarker of the present invention with the additional biomarker mentioned, namely hsa-piR-019324 nucleic acid, improves further specificity and sensitivity of testing.

Furthermore, the method includes embodiments wherein additionally the level or amount of expression of at least miR hsa-miR-15a-5p (SEQ ID No. 3), hsa-miR-425-3p (SEQ ID No. 4), hsa-miR-150-5p (SEQ ID No. 5), hsa-miR-3126-3p (SEQ ID No. 6), hsa-miR-324-5p (SEQ ID No. 7), or hsa-miR-6078 (SEQ ID No. 8) is determined.

The use of at least one of these additional miR improves the specificity and sensitivity of the method according to the present invention further. In an embodiment, the method according to the present invention is a method wherein under-expression of at least one of hsa-piR-019324, hsa-miR-15a-5p, hsa-miR-425-3p, hsa-miR-150-5p, hsa-miR-3126-3p, hsa-miR-324-5p or over-expression of hsa-miR-6078 compared to the expression level or amount in a normal sample is indicative of prostate cancer.

In an embodiment, the methods according to the present invention allow a differential diagnosis between prostate cancer and BPH based on the expression level or amount of hsa-piR-018849 nucleic acid in a sample obtained from a subject having signs of prostate disease, disorder or condition or suspected to suffer from a prostate disease, disorder or condition. Additionally, the level or amount of expression of at least one of the following nucleic acids is determined: hsa-piR-019324, hsa-miR-15a-5p, hsa-miR-425-3p, hsa-miR-150-5p, hsa-miR-3126-3p, hsa-miR-324-5p or hsa-miR-6078.

In another aspect, the present invention relates to a kit for diagnosing prostate cancer or benign prostate disease, disorder or condition in a subject. Namely, said kit comprises means for determining the expression level or amount of at least the hsa-piR-018849 nucleic acid as well as instructions on how to use said test kit for the method according to the present invention. Optionally, the test kit may contain additional means for determining the expression level or amount of at least one of hsa-piR-019324, hsa-miR-15a-5p, hsa-miR-425-3p, hsa-miR-150-5p, hsa-miR-3126-3p, hsa-miR-324-5p or hsa-miR-6078.

The skilled person is well aware of suitable means allowing to determine the level or amount of expression of small RNA molecules including miRNA and piRNA. For example, in a first step a DRNA library is prepared from the total RNA obtained from the sample of said subject. The library preparation is conducted by known means, typically, respective library kits obtainable from different manufacturers.

The libraries were quantified with respective test kits and quality control may be conducted.

Further, the level or amount of the specific nucleic acids is determined e. g. by next generation sequencing or by a PCR-based method. The respective components for effecting next generation sequencing or PCR determination are known to the skilled person and can be obtained from various manufacturers.

Further, the use of the biomarker according to the present invention, namely hsa-piR-018849 nucleic acid, as a marker for differential diagnosis of prostate diseases, disorders or conditions is disclosed. In particular, the present invention relates to the use of this biomarker, hsa-piR-018849, for differential diagnosis of subjects suspected to suffer from prostate cancer and BPH, respectively.

In an embodiment, the biomarker may be supplemented with at least one additional nucleic acid selected from: hsa-piR-019324, hsa-miR-15a-5p, hsa-miR-425-3p, hsa-miR-150-5p, hsa-miR-3126-3p, hsa-miR-324-5p or hsa-miR-6078.

It has been recognized that the biomarker according to the present invention is suitable for non-invasive, fast and reliable use in diagnostics in prostate diseases, disorders and conditions. The biomarker allows differentiation of different prostate disease, disorders or conditions with high specificity and sensitivity.

Further, a method of treatment of prostate cancer with chemotherapy, surgery or by other means is described, said method includes the step of determining the level or amount of expression of hsa-piR-018849 nucleic acid and based on said level or amount of expression of said piR, treating the subject by chemotherapy accordingly. Further, the method of treating includes monitoring the progress of treatment based on the level or amount of expression of the hsa-piR-018849 nucleic acid. In case of a level or amount of expression similar to normal samples, treatment may be finished or suspended. Also, possible relapse of prostate cancer may be monitored based on the level or amount of expression of hsa-piR-018849 nucleic acid whereby treatment is required in case of a decrease of the respective level or amount of expression of said nucleic acid.

The present invention will be described further by way of samples without limiting the same.

### Material and Methods

### Sample Collection

The urine samples of 85 patients were collected at the clinic for Urology at Helios University hospital, Wuppertal. All patients had symptoms of the lower urinary tract and were undergoing a trans-rectal punch biopsy. Exclusion criteria for study participation were inability to actively consent to the study, infectious diseases (e.g. HIV, hepatitis, TB, etc.), urinary diversion using intestinal segments, saliva-reducing underlying diseases, radiotherapy of the pelvis or antiandrogenic therapy in the past. Based on the biopsy results two groups could be formed: Patients with prostate carcinoma (n=53) and patients with benign prostate hyperplasia (n=32). The minimum quantity of urine required for our analysis was met in 53 (25 BPH, 28 PCa) samples. The samples were processed in random order for an equal treatment.

### From Urine to small RNA-Library

The samples were defrosted and aliquoted in a water bath at 2°C. For total RNA-extraction the *Urine microRNA Purification Kit (Cat. 29000,* Norgen Biotek Corp, Thorold, Canada) was used according to the manufacturer's instructions. RNA from uinary exosomes was isolated using the Norgen Urine Exosome RNA Isolation Kit (Cat. 47200, Norgen Biotek Corporation) with a starting volume of 4 ml urine from each sample. The concentration of the preparations was measured using *Qubit Fluorimeter*^{™} *3.0* by Invitrogen Life Technologies Corp., Carlsbad, CA, USA.

The library preparation was done with the *QIAseq miRNA Library Kit* (Qiagen, Hilden, Germany) according to the manufactor's instructions from August 2018. An extended 3' ligation of 18 hours was performed to increase efficiency. In all steps industrially purified nuclease-free water was used, which was treated without DEPC. Apart from this, the manufacturer's instructions and specified concentration ranges were followed precisely. During the cDNA synthesis (reverse transcriptase reaction), unique molecular identifiers (UMI) were integrated which mark the original miRNA input quantity and thus counteract a quantitative bias after the amplification reaction.

The libraries were quantified with the *Qubit Fluorimeter*^{™} 3.0 (Invitrogen Life Technologies Corp., Carlsbad, CA, USA) using the Qubit^{™} microRNA Assay Kit (Cat. Q32881). Quality control was done using polyacrylamide gel electrophoresis (PAGE) and capillary electrophoresis on the *Agilent 2100 Bioanalyzer*^{™} (Kit Agilent DNA 1000, Agilent, Santa Klara, CA, USA).

**Next Generation Sequencing** was performed using Illumina NextSeq 500^{™}. The reading depth was 75 base pairs, which also read the unique molecular identifiers.

### Bioinformatic Analysis

For primary analysis, the *data analysis center* by Qiagen N.V. (qiagen.com) was used. Here the adapters were removed and the original reads adjusted to real numbers using the UMIs. The resulting sequences were mapped with the databases miRBase version 21, Kozomara, A., et. SI., Nucleic acids research 47, D155-D162, doi:10.1093/nar/gky1141 (2018) and Griffiths-Jones, S. Nucleic Acids Res 32, D109-D111, doi:10.1093/nar/gkh023 (2004). miRBase hairpin, mRNAandotherRNA Kozomara, A. & Griffiths-Jones, S. Nucleic acids research 39, D152-D157, doi:10.1093/nar/gkq1027 (2010) and piRNABank, Sai Lakshmi, et. al., Nucleic acids research 36, D173-177, doi:10.1093/nar/gkm696 (2008). The secondary analysis and the following analysis steps and graphics were done using *R version 3.5.3 and 3.6.0,* R-Core-Team. A Language and Environment for Statistical Computing. https://www.r-project.org/ (2019).

As a preprocessing step miRNAs and piRNAs with a variance near or equal to zero or with many zero counts (more than three in each group) were removed. This kept 1795 of 2539 miRNAs and 64 of 265 piRNAs within the total small RNA data set. Equally preprocessed, 115 of 2537 exosomal miRNAs and 63 of 455 exosomal piRNAs remained. The following analysis steps were done separately for the respective miRNA and piRNA dataset.

The samples were split randomly into a training dataset (18 PCA + 15 BPH) and a test dataset (10 PCA + 10 BPH for validation). The *DESeq2 R package version 1.22.2,* Love, M. I., Huber, et. al.,. Genome biology 15, 550, doi:10.1186/s13059-014-0550-8 (2014) was used to normalise the data. Furthermore p-values and fold changes were calculated. In addition, the adjusted p-values (padj) were calculated using the Benjamini-Hochberg procedure to avoid an inflated Type I error rate, Kim, K. I. & van de Wiel, M. A. BMC bioinformatics 9, 114, doi:10.1186/1471-2105-9-114 (2008). Furthermore, the logarithm to base 2 was determined from the fold change values (Log2-Fc).

On the training dataset a statistical analysis was performed with DESeq2 using an adjusted p-value < 0.05 and a fold change > 2 or < 0.5 as a selection criterion in order to obtain biomarker candidates. Each single biomarker candidate was evaluated on the yet unused test dataset using a receiver operating characteristic analysis (ROC). ROC curves and area under the curve (AUC) values were calculated to assess the performance of the biomarker candidates on new data.

In the following analysis we found 105 miRNAs significantly differentially expressed. Thereof 74 were under- and 31 overexpressed. We included 79 miRNAs that had logfc <-1 or >1. For us differentially expressed miRNAs are defined as those that additionally had an adjusted p-value of <0.05. The values in the tables result from the described procedure. In addition, the AUC values of all 79 included candidates were calculated on the test dataset for validation.

**Table 1 differentially expressed miRNA**

| Micro-RNA overexpression PCa vs. BPH | | | | |
|---|---|---|---|---|
| **miRNA** | **Log2-Fold-Change** | **p-value** | **adjusted p-value** | **AUC-value** |
| **hsa-miR-6078** | **1,481** | **2,36E-03** | **2,30E-02** | 0,7 |
| hsa-miR-936 | 1,297 | 8,09E-06 | 4,56E-04 | 0,51 |
| hsa-miR-211-5p | 1,295 | 2,04E-04 | 4,60E-03 | 0,67 |
| hsa-m iR-6729-3p | 1,294 | 3,95E-04 | 6,91 E-03 | 0,44 |
| hsa-miR-891a-5p | 1,275 | 2,01 E-03 | 2,09E-02 | 0,64 |
| hsa-m iR-6080 | 1,172 | 2,50E-03 | 2,39E-02 | 0,64 |
| hsa-miR-3668 | 1,070 | 4,30E-03 | 3,44E-02 | 0,65 |
| hsa-miR-5088-5p | 1,037 | 7,94E-04 | 1,10E-02 | 0,54 |
| hsa-miR-6731-3p | 0,981 | 1,54E-03 | 1,75E-02 | - |
| hsa-miR-4469 | 0,964 | 1,50E-03 | 1,74E-02 | - |
| hsa-miR-3940-3p | 0,962 | 8,00E-03 | 4,83E-02 | - |
| hsa-miR-137 | 0,957 | 7,54E-03 | 4,63E-02 | - |
| hsa-miR-3929 | 0,922 | 3,33E-03 | 2,83E-02 | - |
| hsa-m iR-6790-5p | 0,875 | 2,74E-03 | 2,54E-02 | - |
| hsa-miR-218-5p | 0,856 | 7,07E-03 | 4,52E-02 | - |
| hsa-miR-7108-3p | 0,855 | 2,53E-04 | 5,17E-03 | - |
| hsa-miR-3138 | 0,844 | 8,56E-04 | 1,16E-02 | - |
| hsa-miR-4451 | 0,822 | 5,74E-03 | 3,99E-02 | - |
| hsa-miR-6802-5p | 0,797 | 2,26E-03 | 2,23E-02 | - |
| hsa-miR-6785-3p | 0,791 | 2,12E-03 | 2,17E-02 | - |
| hsa-miR-6863 | 0,726 | 3,23E-03 | 2,82E-02 | - |
| hsa-miR-4501 | 0,724 | 4,80E-03 | 3,55E-02 | - |
| hsa-miR-4712-5p | 0,707 | 4,81 E-04 | 7,75E-03 | - |
| hsa-miR-5196-5p | 0,696 | 7,28E-03 | 4,56E-02 | - |
| hsa-miR-6515-5p | 0,681 | 1,42E-04 | 3,96E-03 | - |
| hsa-miR-218-2-3p | 0,641 | 5,62E-03 | 3,98E-02 | - |
| hsa-miR-6717-5p | 0,636 | 6,28E-03 | 4,18E-02 | - |
| hsa-miR-885-3p | 0,598 | 8,20E-03 | 4,83E-02 | - |
| hsa-miR-4754 | 0,588 | 7,09E-03 | 4,52E-02 | - |
| hsa-miR-6820-3p | 0,584 | 8,05E-03 | 4,83E-02 | - |
| hsa-miR-296-3p | 0,437 | 2,96E-03 | 2,62E-02 | - |

| Micro-RNA underexpression PCa vs. BPH | | | | |
|---|---|---|---|---|
| **miRNA** | **Log2-Fold-Change** | **p-value** | **adjusted p-value** | **AUC-value** |
| hsa-miR-451a | -4,916 | 1,60E-09 | 9,79E-07 | 0,57 |
| hsa-miR-27a-3p | -3,352 | 1,17E-06 | 1,03E-04 | 0,52 |
| hsa-miR-106b-5p | -3,023 | 2,49E-07 | 3,81 E-05 | 0,52 |
| hsa-miR-151b/151a-5p | -2,940 | 4,05E-07 | 4,13E-05 | 0,54 |
| hsa-miR-182-5p | -2,937 | 3,59E-07 | 4,13E-05 | 0,58 |
| hsa-miR-6865-5p | -2,860 | 1,04E-08 | 3,19E-06 | 0,53 |
| hsa-miR-16-2-3p | -2,565 | 7,09E-05 | 2,55E-03 | 0,51 |
| hsa-miR-17-5p | -2,385 | 6,13E-04 | 9,16E-03 | 0,53 |
| hsa-miR-200c-3p | -2,369 | 1,27E-04 | 3,71 E-03 | 0,54 |
| hsa-m iR-200b-3p | -2,334 | 3,21 E-04 | 5,98E-03 | 0,54 |
| hsa-miR-142-5p | -2,228 | 5,87E-03 | 3,99E-02 | 0,46 |
| hsa-miR-20a-5p | -2,177 | 3,23E-04 | 5,98E-03 | 0,52 |
| **hsa-miR-15a-5p** | **-2,175** | **1,29E-03** | **1,51 E-02** | **0,71** |
| hsa-miR-320a | -2,163 | 1,76E-04 | 4,49E-03 | 0,59 |
| hsa-miR-146b-5p | -2,092 | 2,23E-04 | 4,70E-03 | 0,55 |
| **hsa-mi R-3126-3p** | **-2,057** | **1,12E-07** | **2,28E-05** | **0,76** |
| hsa-miR-126-3p | -2,002 | 1,95E-04 | 4,60E-03 | 0,55 |
| hsa-miR-149-5p | -2,000 | 8,20E-06 | 4,56E-04 | 0,48 |
| hsa-m iR-26a-2-3p | -1,973 | 1,67E-05 | 7,77E-04 | 0,50 |
| hsa-miR-363-3p | -1,964 | 1,21 E-04 | 3,69E-03 | 0,43 |
| hsa-miR-196b-5p | -1,960 | 4,75E-04 | 7,75E-03 | 0,47 |
| hsa-miR-375 | -1,937 | 3,65E-03 | 3,04E-02 | 0,51 |
| hsa-miR-20b-5p | -1,932 | 1,28E-05 | 6,55E-04 | 0,50 |
| hsa-miR-28-5p | -1,909 | 1,86E-04 | 4,55E-03 | 0,54 |
| hsa-miR-141-5p | -1,880 | 7,82E-06 | 4,56E-04 | 0,51 |
| hsa-miR-103a-3p | -1,861 | 1,18E-03 | 1,45E-02 | 0,53 |
| hsa-miR-652-3p | -1,852 | 9,03E-04 | 1,18E-02 | 0,57 |
| hsa-miR-320b | -1,838 | 5,44E-04 | 8,32E-03 | 0,49 |
| hsa-miR-183-5p | -1,822 | 3,17E-04 | 5,98E-03 | 0,61 |
| hsa-miR-22-3p | -1,821 | 1,81 E-03 | 1,95E-02 | 0,49 |
| hsa-miR-148a-3p | -1,776 | 4,87E-03 | 3,55E-02 | 0,47 |
| hsa-miR-103b | -1,759 | 3,62E-04 | 6,52E-03 | 0,63 |
| hsa-miR-151a-3p | -1,754 | 2,63E-03 | 2,47E-02 | 0,57 |
| hsa-miR-28-3p | -1,723 | 4,73E-04 | 7,75E-03 | 0,52 |
| hsa-miR-195-5p | -1,706 | 2,10E-04 | 4,60E-03 | 0,58 |
| hsa-miR-7641 | -1,688 | 5,87E-06 | 4,49E-04 | 0,51 |
| hsa-m iR-200b-5p | -1,677 | 1,16E-03 | 1,45E-02 | 0,53 |
| hsa-miR-409-3p | -1,673 | 1,78E-05 | 7,77E-04 | 0,45 |
| hsa-miR-107 | -1,647 | 1,73E-03 | 1,93E-02 | 0,47 |
| hsa-miR-423-5p | -1,645 | 4,12E-03 | 3,36E-02 | 0,58 |
| hsa-let-7g-5p | -1,596 | 4,40E-03 | 3,45E-02 | 0,51 |
| hsa-miR-671-5p | -1,583 | 3,12E-05 | 1,27E-03 | 0,56 |
| hsa-miR-93-3p | -1,568 | 1,69E-04 | 4,49E-03 | 0,64 |
| hsa-miR-96-5p | -1,536 | 4,32E-03 | 3,44E-02 | 0,56 |
| hsa-miR-574-5p | -1,534 | 1,06E-03 | 1,35E-02 | 0,45 |
| hsa-miR-155-5p | -1,523 | 3,33E-03 | 2,83E-02 | 0,61 |
| hsa-m iR-200a-5p | -1,505 | 9,12E-05 | 2,94E-03 | 0,51 |
| hsa-miR-510-3p | -1,496 | 4,67E-03 | 3,55E-02 | 0,50 |
| hsa-miR-98-5p | -1,477 | 3,68E-03 | 3,04E-02 | 0,50 |
| **hsa-mi R-324-5p** | **-1,404** | **7,60E-04** | **1,08E-02** | **0,74** |
| hsa-miR-7-5p | -1,384 | 2,92E-03 | 2,62E-02 | 0,63 |
| **hsa-mi R-150-5p** | **-1,378** | **2,84E-03** | **2,59E-02** | **0,76** |
| hsa-miR-135b-5p | -1,376 | 5,57E-03 | 3,98E-02 | 0,62 |
| hsa-miR-19b-3p | -1,345 | 8,17E-03 | 4,83E-02 | 0,62 |
| hsa-miR-1343-3p | -1,335 | 8,96E-05 | 2,94E-03 | 0,41 |
| hsa-miR-145-5p | -1,293 | 5,65E-03 | 3,98E-02 | 0,68 |
| hsa-miR-429 | -1,291 | 6,57E-03 | 4,32E-02 | 0,58 |
| hsa-miR-744-5p | -1,173 | 6,21 E-03 | 4,18E-02 | 0,56 |
| hsa-miR-503-5p | -1,164 | 5,36E-04 | 8,32E-03 | 0,65 |
| hsa-miR-663a | -1,160 | 6,30E-04 | 9,18E-03 | 0,61 |
| hsa-miR-6081 | -1,139 | 2,23E-03 | 2,23E-02 | 0,64 |
| hsa-miR-5100 | -1,105 | 1,28E-03 | 1,51 E-02 | 0,59 |
| **hsa-mi R-425-3p** | **-1,103** | **8,91 E-04** | **1,18E-02** | **0,71** |
| hsa-miR-636 | -1,095 | 4,53E-03 | 3,51 E-02 | 0,66 |
| hsa-miR-6877-5p | -1,084 | 5,12E-05 | 1,96E-03 | 0,61 |
| hsa-miR-664a-5p | -1,081 | 5,81 E-03 | 3,99E-02 | 0,56 |
| hsa-miR-106b-3p | -1,078 | 7,30E-03 | 4,56E-02 | 0,48 |
| hsa-miR-7977 | -1,069 | 1,94E-03 | 2,05E-02 | 0,64 |
| hsa-miR-4793-5p | -1,047 | 1,82E-03 | 1,95E-02 | 0,52 |
| hsa-miR-3922-5p | -1,029 | 4,88E-03 | 3,55E-02 | 0,64 |
| hsa-miR-2277-3p | -1,002 | 7,56E-03 | 4,63E-02 | 0,60 |
| hsa-miR-324-3p | -0,977 | 6,98E-03 | 4,52E-02 | - |
| hsa-miR-3907 | -0,961 | 4,87E-03 | 3,55E-02 | - |
| hsa-miR-1301-3p | -0,746 | 8,45E-03 | 4,92E-02 | - |

In addition to miRNAs, other small RNAs were determined through the extraction procedure used in this study. Due to their comparable size, they were also isolated using the filter-based method. The databases used identified piRNAs, which also showed significant differences between the two groups. The data were processed and presented as described for miRNAs.

**Table 2 differentially expressed piRNA**

| piRNA Overexpression | | | | |
|---|---|---|---|---|
| **piRNa** | **Log2-Fold-Change** | **p-value** | **adjusted p-value** | **AUC-value** |
| hsa_piR_020401 | 1,259 | 2,81 E-03 | 3,59E-02 | 0,64 |

| piRNA Underexpression | | | | |
|---|---|---|---|---|
| **piRNa** | **Log2-Fold-Change** | **p-value** | **adjusted p-value** | **AUC-value** |
| hsa_piR_001318 | -2,110 | 1,25E-05 | 5,32E-04 | 0,56 |
| hsa_piR_018573 | -1,945 | 2,05E-05 | 5,32E-04 | 0,61 |
| **hsa_piR_018849** | **-2,292** | **2,49E-05** | **5,32E-04** | **0,88** |
| **hsa_piR_019324** | **-2,032** | **4,68E-05** | **7,49E-04** | **0,72** |

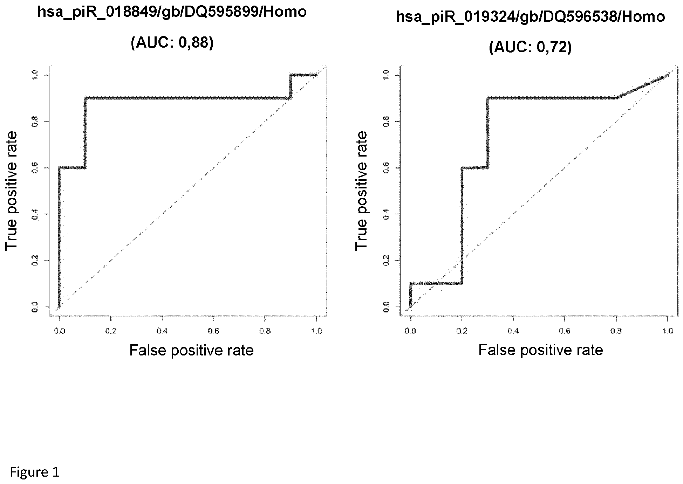

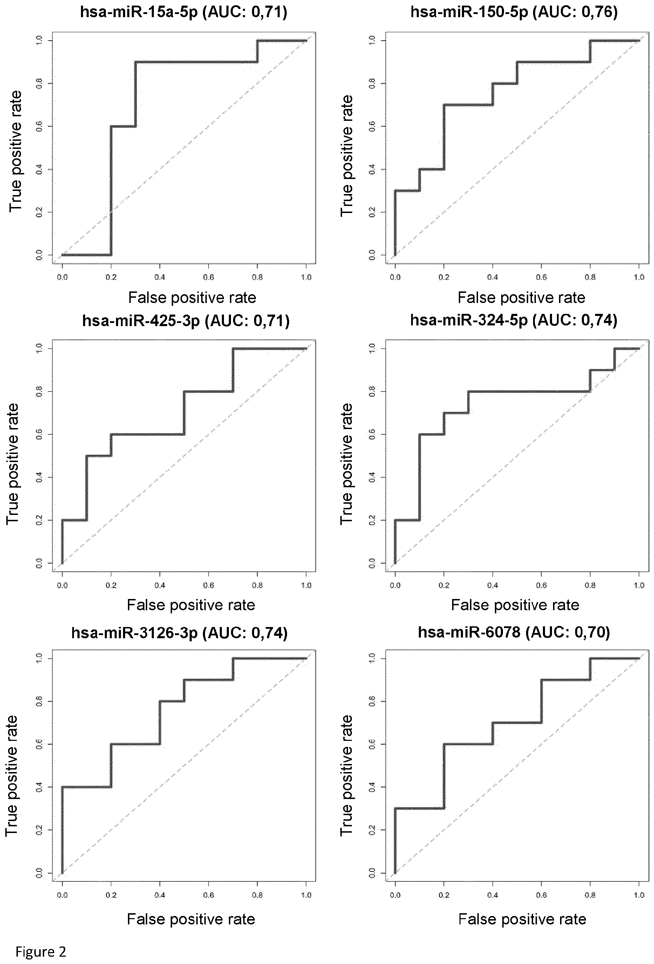

The results of the statistical comparison of the two randomly generated groups were finally validated on the data set of the remaining samples (test group = 10 BPH vs. 10 PCa). This is shown graphically by ROC curves in Fig. 1 and 2 All candidates with an adjusted p-value of < 0.05 and a Log2-Fold-Change of >2 and <0.5 were examined. False positive rates and true positive rates were compared on the axes. The samples that achieved values from 0.7 upwards were considered valid. This resulted in 6 miRNAs (has-miR-15a-5p, has-miR-3126-3p, has-miR-324-5p, has-miR-150-5p, has-miR-425-3p, has-miR-6078) and 2 piRNAs (has_piR_018849, has_piR_019324) exceeding the set target of 70% area under the curve (AUC, integral), see Fig. 2.
For the piRNAs examined here, the validation confirmed the following two candidates: hsa_piR_018849, hsa_piR_019324. They were compared with entries of the piRBase database51. For piR_019324 no studies on function and interaction were found. For piR_018849, 95 reference sequences could be specified, but no target gene. Therefore, their molecular function still seems to be in need of clarification. For diagnostic studies, however, they could already be noted. The results of piR_018849 show a clear fold change value and robust significance and were validated with a very precise 90% specificity/sensitivity.

## Claims

1. A method for differential diagnosis or for identification of prostate cancer in a subject suspected to suffer from prostate cancer or other type of prostate disease, disorder or condition, comprising the steps of:
a) providing a sample of said subject to be diagnosed;
b) determining the level or amount of the expression of hsa-piR-018849 nucleic acid in said sample;
c) differential diagnosing prostate cancer or other type of prostate disease, disorder or condition, in particular, a benign prostate disease, disorder or condition or identifying prostate cancer based on the level or amount of the hsa-piR-018849 nucleic acid determined in step b).

2. A method for screening for prostate cancer in a subject comprising the steps of
a) providing a sample of said subject;
b) determining the level or amount of the expression of hsa-piR-018849 nucleic acid in said sample;
c) comparing said level or amount of the expression of hsa-piR-018849 nucleic acid to a reference value from a normal sample(s) whereby a decrease of the expression level or amount is indicative of prostate cancer.

3. A method for screening for benign prostatic hyperplasia (BPH) in a subject suspected to suffer from a prostate disease, disorder or condition, comprising the steps of
a) providing a sample of said subject;
b) determining the level or amount of the expression of hsa-piR-018849 nucleic acid in said sample;
c) comparing said level or amount of the expression of hsa-piR-018849 nucleic acid determined in step b) to a reference value and excluding prostate cancer or other malignant prostate diseases, disorders or conditions.

4. A method for monitoring the development, progress or lapse of prostate cancer in a subject afflicted with, suspected to be afflicted with or having a risk of developing prostate cancer, comprising the steps of
a) determining the level or amount of expression of hsa-piR-018849 nucleic acid in a sample of said subject at a first time point, and
b) optionally, determining the level or amount of said nucleic acid, at a second time point, and
c) comparing the level or amount of expression of said nucleic acid determined in step a) to the level or amount of said nucleic acid detected in step b) or to a reference value of a normal sample(s).

5. A method for the stratification of the therapeutic regimen of a subject being afflicted with, suspected to be afflicted with or being at risk of developing prostate cancer, comprising the steps of
a) determining the level or amount of expression of hsa-piR-018849 nucleic acid in a sample of said subject, and
b) determining a therapeutic regimen of said subject taking into account or based on that level or amount of the expression of hsa-piR-018849 nucleic acid relative to the level or amount of the expression of hsa-piR-018849 nucleic acid in a normal sample.

6. The method of any one of the preceding claims where a low level or amount of expression of hsa-piR-018849 nucleic acid in said sample of a subject compared to a normal sample providing a control relative to the test sample of said subject, is indicative for being afflicted with prostate cancer or being at risk of developing prostate cancer or for the risk of developing relapse of prostate cancer.

7. The method according to any one of the preceding claims wherein the sample of the patient is a sample of body fluid or body tissue of said patient, in particular, of urine or blood, like whole blood, serum or plasma, like a sample from urine.

8. The method according to any one of the preceding claims wherein the subject is a human.

9. The method according to any one of the preceding claims further comprising determining the level or amount of expression of hsa-piR-019324 nucleic acid.

10. The method according to any one of the preceding claims further comprising determining the level or amount of expression of at least one of the miR: hsa-miR-15a-5p, hsa-miR-425-3p, hsa-miR-150-5p, hsa-miR-3126-3p, hsa-miR-324-5p or hsa-miR-6078.

11. The method according to any one of the claims 9 to 10 wherein under-expression of at least one of hsa-piR-019324, hsa-miR-15a-5p, hsa-miR-425-3p, hsa-miR-150-5p, hsa-miR-3126-3p, hsa-miR-324-5p or over-expression of hsa-miR-6078 compared to the expression level or amount in a normal sample is indicative of prostate cancer.

12. Test kit for diagnosing prostate cancer or benign prostate disease, disorder or condition in a subject, said kit comprises means for determining the expression level or amount of at least hsa-piR-018849 and, optionally, further means for determining the expression level or amount of at least one of hsa-piR-019324, hsa-miR-15a-5p, hsa-miR-425-3p, hsa-miR-150-5p, hsa-miR-3126-3p, hsa-miR-324-5p or hsa-miR-6078 and instructions on how to use said test kit for a method according to any one of the claims 1 to 11.

13. A test kit according to claim 12 whereby the kit is a kit for next generation sequencing or a PCR kit.

14. An use of hsa-piR-018849 nucleic acid as a marker for differential diagnosis of prostate disease, disorders or conditions in a subject.

15. The use of hsa-piR-018849 nucleic acid according to claim 14 for differential diagnosis of prostate cancer and benign prostatic hyperplasia (BPH).

16. The use of hsa-piR-018849 nucleic acid according to claim 14 or 15 in combination with at least one of hsa-piR-019324, hsa-miR-15a-5p, hsa-miR-425-3p, hsa-miR-150-5p, hsa-miR-3126-3p, hsa-miR-324-5p or hsa-miR-6078.


SEQUENCE LISTING

<110>  Private Universitaet Witten/Herdecke gGmbH

<120>  Method for differential diagnosis of prostate disease and marker
       for differential diagnosis of prostate disease as well as kit
       therefor

<130>  6636-0001 EP-1

<160>  8

<170>  PatentIn version 3.5

<210>  1
<211>  29
<212>  DNA
<213>  Homo sapiens

<400>  1
tgggaggaag ctcaccccgg atcttgagg                                          29


<210>  2
<211>  30
<212>  DNA
<213>  homo sapiens

<400>  2
tggggaagtg tcacagatgt gcacatgggc                                         30


<210>  3
<211>  22
<212>  RNA
<213>  homo sapiens

<400>  3
uagcagcaca uaaugguuug ug                                                 22


<210>  4
<211>  22
<212>  RNA
<213>  homo sapiens

<400>  4
aucgggaaug ucguguccgc cc                                                 22


<210>  5
<211>  22
<212>  RNA
<213>  homo sapiens

<400>  5
ucucccaacc cuuguaccag ug                                                 22


<210>  6
<211>  22
<212>  RNA
<213>  homo sapiens

<400>  6
caucuggcau ccgucacaca ga                                                22

<210>  7
<211>  22
<212>  RNA
<213>  homo sapiens

<400>  7
   cgcauccccu agggcauugg ug                                                  22

<210>  8
<211>  19
<212>  RNA
<213>  homo sapiens

<400>  8
ccgccugagc uagcugugg                                                    19